# EUROPEAN PATENT APPLICATION

(11) **EP 2 561 821 A1**
(43) Date of publication of application: **27.02.2013**
(21) Application number: 12002358.5
(22) Date of filing: 30.03.2012
(51) Int. Cl.: A61B 19/00

(54) **Tool positioning system**

(30) Priority: 25.08.2011 IN MA29182011
(71) Applicant: Perfint Healthcare Private Limited, Chennai 600017 TN (IN)
(72) Inventor: Velusamy, Gnanasekar, Kurinjipadi 607302 Tamil Nadu (IN)
(74) Representative: Bradley, Adrian

(57) **Abstract**

A tool positioning system adapted for use with an imagine system is provided. The tool positioning system is configured to accurately position a guide, the guide being suitable for inserting an interventional tool from an insertion point to a target point inside a patient. The s tool positioning system is configured to be docked at a pre-defined docking position along a movable patient cradle of the imaging system. The tool positioning system includes standalone device and a robotic positioner. The robotic positioner is coupled to the standalone device and configured to grip and move the guide to a desired position. The system further includes a movement sensing module coupled to the standalone device and configured to calculate movement of the patient from breathing and processing circuitry configured to determine the pre-defined docking position and the desired position.

## Description

### BACKGROUND

The invention relates generally to medical diagnostic imaging systems, and more specifically to a tool positioning system adapted for use with medical imaging systems.

In order to perform various procedure accurately, it is often required to position a tool appropriately with respect to the anatomy of the subject. On positioning the tool accurately, a physician, may insert the tool into the subject to obtain a sample of, for example, a tissue, bone, fluid, etc. This sample tissue is used particularly in the diagnosis and treatment of potentially cancerous tumors, pre-malignant conditions, and other diseases or disorders. Typically, in the case of tumors, when the physician suspects that cancer or an otherwise diseased condition exists, a biopsy is performed to determine if in fact cells from the tumor are cancerous or otherwise diseased. Many biopsies, such as percutaneous biopsies, are performed with a needle-like instrument used to collect the tissue for analysis.

In recent years, the performance of interventional medical procedures such as needle biopsies has been enhanced by the use of several imaging techniques such as x-ray imaging, CT scans, continuous CT (CCT), magnetic resonance imaging (MRI), fluoroscopy, single photon emission CT (SPECT), positron emission tomography (PET), and the like. The imaging equipment allows a radiologist, surgeon, physician, or other medical personnel, to plan and track the percutaneous insertion of interventional devices, such as biopsy needles, in a subject during diagnostic and/or therapeutic procedures.

Several surgical instrument guidance devices have been proposed for use in conjunction with imaging systems to allow a user to accurately place an interventional tool within a patient's body. For example, U.S. Pat. No. 4,583,538 proposes a free standing biopsy guide that is adapted to hold needles or probes at various selectable calculated angles. In using the device proposed in that patent, a reference point on the patient's body is found that exactly correlates to a point on the CT scan. This is accomplished by means of a localization device placed on the patient's skin which can be identified in cross section on the CT scan. Measurements of the localization device on the CT scan are then correlated to the device on the patient. The free standing biopsy guide is then adjusted according to those calculations. One disadvantage of the device taught by this patent is the time required to correlate the patient body reference point with selected points on the CT scan. In addition, certain inaccuracies may be introduced during the point correlation step and while adjusting the free standing guidance device. Accordingly, it would be desirable to provide a biopsy or other surgical instrument guide that is affixed in a known position relative to the CT scanner apparatus whereby precise and automatic correlation between the coordinate systems of the guidance device, patient table, and patient image volume are automatically established.

U.S. Pat. No. 6,853,856 describes a medical imaging system for conducting an image-guided medical procedure on a subject, the system comprising: a medical imaging apparatus for obtaining volumetric images of the subject; means for planning an interventional procedure on a subject using the volumetric images; a mechanical arm assembly disposed in proximity to the medical imaging apparatus, the mechanical arm assembly comprising a base support, a distal end, a plurality of arm segments, and a plurality of joints between the arm segments for carrying out the interventional procedure; and an end-effector disposed at the distal end of the mechanical arm assembly, the end-effector comprising gripping means for selectively gripping a surgical instrument during the interventional procedure with a gripping force ranging from zero to a force which prevents relative movement between the gripping means and the surgical instrument wherein the end-effector further comprises; a first finger portion having a first gripping surface; a second finger portion having a second gripping surface, the first and second gripping surfaces being opposed to one another for applying a gripping force to the surgical instrument; a first surgical instrument guide disposed on the first finger portion and extending towards the second finger portion; and a second surgical instrument guide disposed on the second finger portion end extending towards the first finger portion.

U.S. Pat. App. No.20060149147 describes a guide apparatus for use with an associated imaging device to direct movement of an associated interventional implement relative to a patient disposed on the imaging device. The guide apparatus includes a connector portion coupling the guide apparatus with the associated imaging device. A main body portion of the apparatus is supported relative to the associated imaging device by the connector portion. A gripping area is formed at a first end of the main body portion, the gripping area being adapted to the guide apparatus for manual gripping by an associated operator. A holding area is formed at a second end of the main body portion. The holding area is adapted to hold the associated interventional implement in an orientation suitable for motion relative to said patient along a selected linear path and also operative to translate the associated interventional implement along said selected linear path (P) in response to manual force applied by the associated human operator at said gripping area. A robot arm positions the apparatus to hold a biopsy needle relative to the patient based on a virtual planned trajectory. A linear slider joint constrains movement of the needle to the path (P) and permits manual insertion of the needle and attendant tactile feedback.

The above describe techniques have several drawbacks. For example, the technique described in the '856 patent required operating the apparatus in fluoroscopy mode to locate the entry point on the patient. In addition, tracking devices to track the position of the patient with respect to the interventional tool. The imaging apparatus needs to be modified to operate the guide apparatus. In some of the techniques described above, permanent fixing of robotic system either on the floor or on to the CT system is required. This may hinder normal usage of the imaging system for other non-interventional procedures. In addition, the techniques described above do not compensate for the movement of targets due to breathing and patient movement, which affect the accuracy of positioning.

The present invention provides a new tool positioning system that overcomes the above-referenced problems and others.

### BRIEF DESCRIPTION

Briefly, according to one embodiment of the invention a medical imaging system for conducting an image guided procedure on a subject is provided. The medical imaging system comprises an imaging apparatus configured to generate volumetric images of a region of interest within the subject, wherein the medical imaging apparatus includes a movable patient cradle. The medical imaging system further includes a tool positioning system configured to be moved to a desired position along the movable patient cradle of the imaging system and further configured to be referenced to the imaging system. The tool positioning system includes a standalone device and a robotic positioner coupled to the standalone device and configured to grip an interventional tool guide and move the tool guide to a desired position and a movement sensing module coupled to the standalone device and configured to calculate movement of the patient

In another embodiment, a tool positioning system adapted for use with an imaging system is provided. The tool positioning system is configured to accurately position a guide, the guide being suitable for inserting an interventional tool from an insertion point to a target point inside a patient. The tool positioning system is configured to be docked at a pre-defined docking position along a movable patient cradle of the imaging system. The tool positioning system includes a standalone device and a robotic positioner coupled to the standalone device and configured to grip and move the guide to a desired position. The system further includes a movement sensing module coupled to the standalone device and configured to calculate movement of the patient from breathing and processing circuitry configured to determine the pre-defined docking position and the desired position.

### DRAWINGS

These and other features, aspects, and advantages of the present invention will become better understood when the following detailed description is read with reference to the accompanying drawings in which like characters represent like parts throughout the drawings, wherein:

FIG. 1 is a schematic view of an exemplary imaging system, in this case a computed tomography ("CT") imaging system, designed to implement the enhanced image data storage scheme in accordance with an exemplary embodiment of the present technique;

FIG. 2 and FIG 3 are diagrammatic views of a tool positioning system used in conjunction with an imaging system implemented according to aspects of the present technique;

FIG. 4 is a diagrammatic view of the tool positioning system implemented according to aspects of the present technique;

FIG. 5A is a diagrammatic view of one embodiment of a tool positioning system in an unlocked position;

FIG. 5B is a diagrammatic view of one embodiment of a tool positioning system in an locked position; and

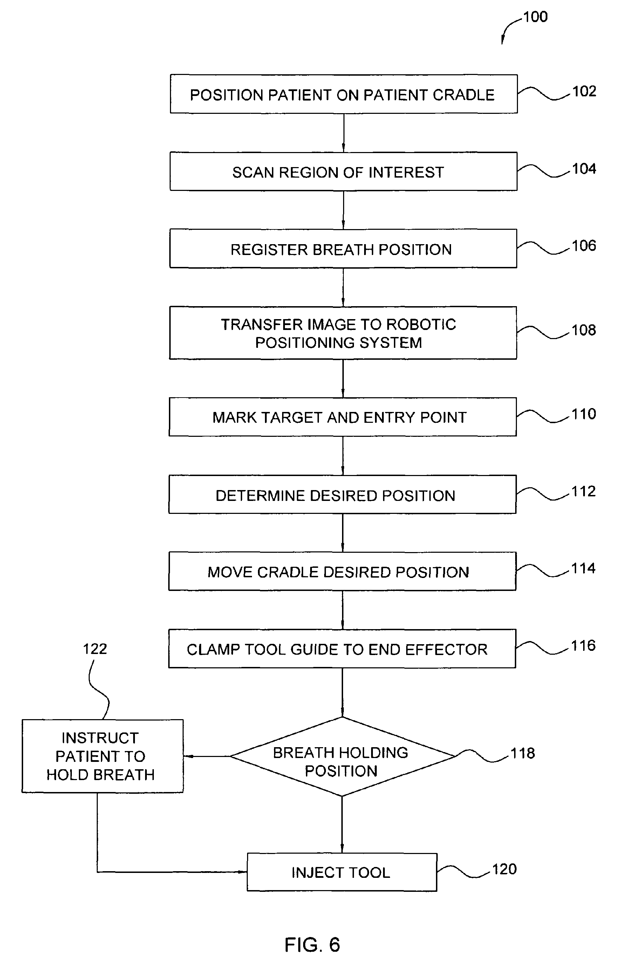

FIG. 6 is a flow chart depicting a method for performing an interventional procedure according to aspects of the present technique

### DETAILED DESCRIPTION

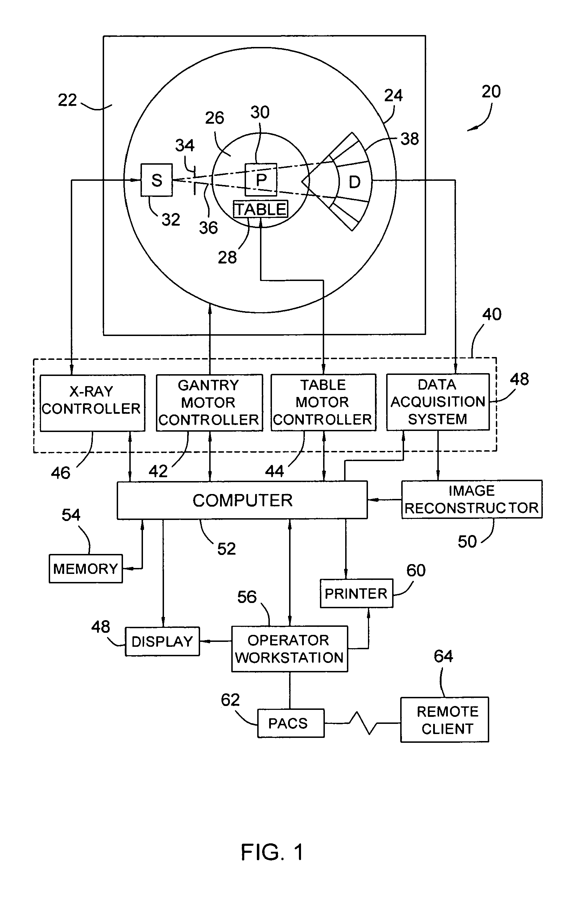

Turning now to the drawings, and referring first to FIG. 1, the present invention will be described as it might be applied in conjunction with an exemplary imaging system, in this case a computed tomography (CT) imaging system. In general, however, it should be borne in mind that the present techniques may be used with image data produced by any suitable imaging modality. The illustrated embodiment of the CT imaging system 20 has a frame 22, a gantry 24, and an aperture (imaging volume or CT bore volume) 26. A patient table 28 is positioned in the aperture 26 of the frame 22 and the gantry 24. The patient table 28 is adapted so that a patient 30 may recline comfortably during the examination process.

The illustrated embodiment of the CT imaging system 20 has an X-ray source 32 positioned adjacent to a collimator 34 that defines the size and shape of the X-ray beam 36 that emerges from the X-ray source 32. In typical operation, the X-ray source 32 projects a stream of radiation (an X-ray beam) 36 towards a detector array 38 mounted on the opposite side of the gantry 24. All or part of the X-ray beam 36 passes through a subject, such as a patient 30, prior to impacting the detector array 38. It should be noted that all or part of the X-ray beam 36 may traverse a particular region of the patient 30, such as the liver, pancreas, heart, and so on, to allow a scan of the region to be acquired. The detector array 38 may be a single slice detector or a multi-slice detector and is generally formed by a plurality of detector elements. Each detector element produces an electrical signal that represents the intensity of the incident X-ray beam 36 at the detector element when the X-ray beam 36 strikes the detector array 38. These signals are acquired and processed to reconstruct an image of the features within the patient 30.

The gantry 24 may be rotated around the patient 30 so that a plurality of radiographic views may be collected along an imaging trajectory described by the motion of the X-ray source 32 relative to the patient 30. In particular, as the X-ray source 32 and the detector array 38 rotate along with the gantry 24, the detector array 38 collects photons resulting from X-ray beam attenuation at the various view angles relative to the patient 30 and produces signals or data representative of the incident photons. Data collected from the detector array 38 then undergoes pre-processing and filtering to condition the data to represent the line integrals of the attenuation coefficients of the scanned patient 30. The processed data, commonly called projections, are then filtered and back projected to formulate an image of the scanned area. Thus, an image or slice is acquired which may incorporate, in certain modes, less or more than 360 degrees of projection data, to formulate an image.

Rotation of the gantry 24 and operation of the X-ray source 32 are controlled by a system controller 40, which furnishes both power and control signals for CT examination sequences. Moreover, the detector array 38 is coupled to the system controller 40, which commands acquisition of the signals generated in the detector array 38. The system controller 40 may also execute various signal processing and filtration functions, such as for initial adjustment of dynamic ranges, interleaving of digital image data, and so forth. In general, system controller 40 commands operation of the imaging system 20 to execute examination protocols and to process acquired data. In the present context, system controller 40 also includes signal processing circuitry, typically based upon a general purpose or application-specific digital computer, associated memory circuitry for storing programs and routines executed by the computer, as well as configuration parameters and image data, interface circuits, and so forth. The system controller 40 includes a gantry motor controller 42 that controls the rotational speed and position of the gantry 24 and a table motor controller 44 that controls the linear displacement of the patient table 28 within the aperture 26. In this manner, the gantry motor controller 42 rotates the gantry 24, thereby rotating the X-ray source 32, collimator 34 and the detector array 38 one or multiple turns around the patient 30. Similarly, the table motor controller 44 displaces the patient table 28, and thus the patient 30, linearly within the aperture 26. Additionally, the X-ray source 32 may be controlled by an X-ray controller 46 disposed within the system controller 40. Particularly, the X-ray controller 46 may be configured to provide power and timing signals to the X-ray source 32.

In the illustrated embodiment, the system controller 40 also includes a data acquisition system 48. In this exemplary embodiment, the detector array 38 is coupled to the system controller 40, and more particularly to the data acquisition system 48. The data acquisition system 48 typically receives sampled analog signals from the detector array 38 and converts the data to digital signals for subsequent processing. An image reconstructor 50 coupled to the computer 52 may receive sampled and digitized data from the data acquisition system 48 and performs high-speed image reconstruction. Alternatively, reconstruction of the image may be done by the computer 52. Once reconstructed, the image produced by the imaging system 10 reveals internal features of the patient 30.

The data collected by the data acquisition system 48, or the reconstructed images, may be transmitted to the computer 52 and to a memory 54. It should be understood that any type of memory to store a large amount of data may be utilized by such an exemplary imaging system 10. Also the computer 52 may be configured to receive commands and scanning parameters from an operator via an operator workstation 56 typically equipped with a keyboard and other input devices. An operator may control the CT imaging system 20 via the operator workstation 56. Thus, the operator may observe the reconstructed image and other data relevant to the system from computer 52, initiate imaging, and so forth.

The CT imaging system 20 also has a display 58 that is coupled to the operator workstation 56 and the computer 52 and may be utilized by a user to observe the reconstructed image, as well as to provide an interface for control of the operation of the CT imaging system 20. In this embodiment, a printer 60 is present to enable a hard copy of a medical image to be printed. In the illustrated embodiment, the CT imaging system 20 is coupled to a picture archiving and communications system (PACS) 62 via the operator workstation 56 for long-term storage of image data. It should be noted that the PACS 62 may be coupled to a remote system 64, such as radiology department information system (RIS), hospital information system (HIS) or to an internal or external network, so that others at different locations may gain access to the image and to the image data. However, access to the image data may also be obtained remotely through the PACS 62.

It should be further noted that the computer 52 and operator workstation 56 may be coupled to other output devices, such as a standard or special purpose computer monitor and associated processing circuitry. One or more operator workstations 56 may be further linked in the CT imaging system 20 for outputting system parameters, requesting examinations, viewing images, and so forth. In general, displays, printers, workstations, and similar devices supplied within the CT imaging system 20 may be local to the data acquisition components, or may be remote from these components, such as elsewhere within an institution or hospital, or in an entirely different location, linked to the imaging system CT via one or more configurable networks, such as the Internet, virtual private networks, wireless networks, and so forth.

As noted above, it should be borne in mind that the CT system referred to herein is merely one exemplary source of image data that may be handled in accordance with the present techniques. Most such systems will include operator interfaces and software specifically adapted to acquire image data and to at least partially process the data in accordance with the specific physics of the imaging modality. Indeed, other arrangements of CT systems, other reconstruction techniques, and so forth may give rise to image data that may be managed as described herein.

The CT system described above is used in conjunction with a tool positioning system to enable a physician, radiologist and other trained medical personnel to accurately positioning a device, such as a tool guide. The tool guide is suitable for inserting an interventional tool such as a needle, from an insertion point to a target point inside a patient. The insertion point and the target point can be derived using the images generated using an imaging system, such as the CT system described in FIG. 1.

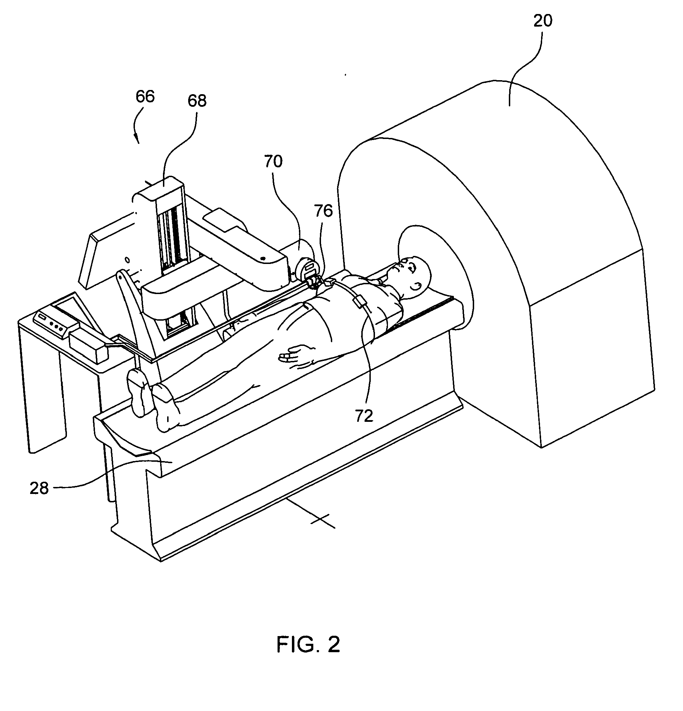

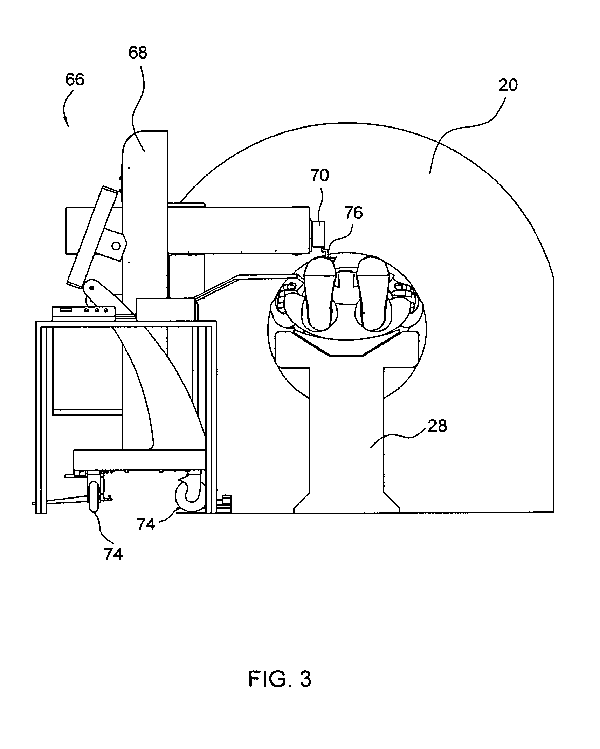

FIG. 2 and FIG. 3 are two views of a tool positioning system adapted for use in an imaging system, according to aspects of the present invention. The tool positioning system 66 includes a standalone device 68, a robotic positioner 70, a patient breath monitoring module 72 and a patient movement restriction bed 78. Each component is described in detail is described in further detail below.

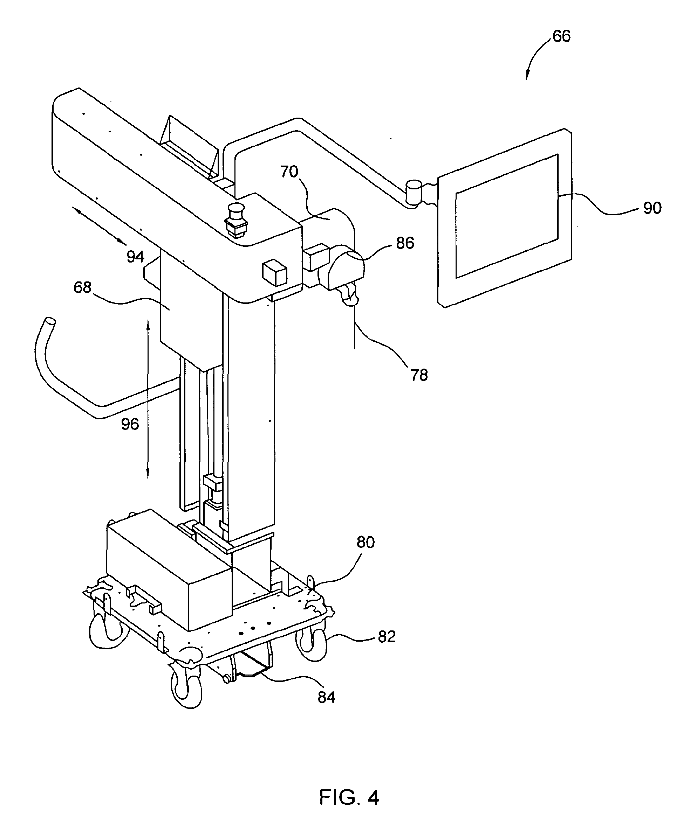

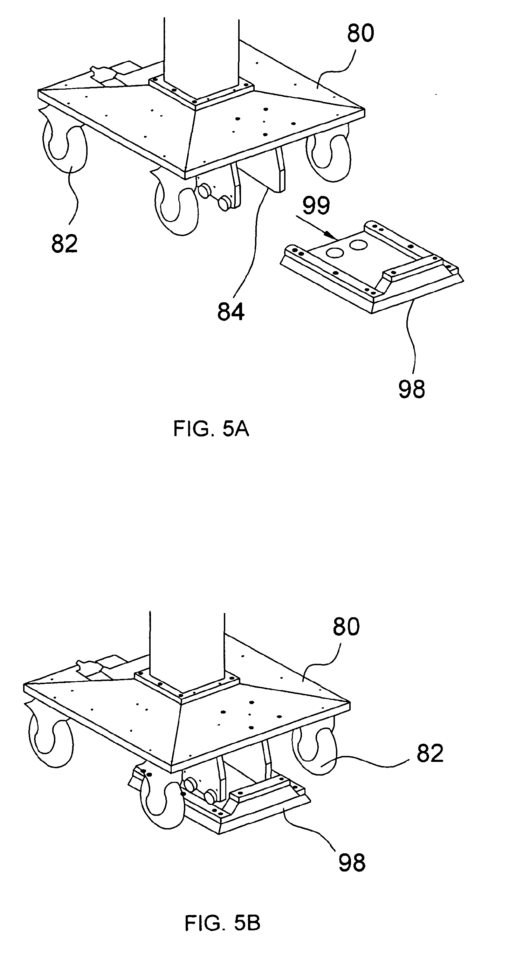

The tool positioning system 66 is configured to be docked at a pre-defined docking position 74 (as shown in FIG. 3) along the movable patient cradle 28 of the CT system 20. The tool positioning system includes a standalone device 68 is locked at point 74 adjacent to the movable patient cradle 28. Robotic positioner 70 is coupled to the standalone device 68 and configured to grip and move an interventional tool guide 76 to a desired position. The desired position is calculated by integrated processing system 90 (not shown). The integrated processing system may include processing circuitry, control circuitry, display means, etc. The standalone device and the robotic positioner will be described in further detail in FIG. 4, FIG. 5A and FIG. 5B.

In one embodiment, the desired position is calculated based on the insertion point and the target point, pre-defined docking position, coordinates derived from the location of the standalone device with respect to the patient cradle. In a further embodiment, the desired position is calculated by accounting for the movement of the patient including from breathing.

The integrated processing system 90 is further configured to determine a distance to move the movable patient cradle of the imaging system such that the patient is outside an imaging space of the imaging system while computing the position of the needle guide, as can be seen in FIG. 2.

Movement sensing module 72 is coupled to the standalone device and configured to calculate movement of the patient from breathing. The calculated movement is used to determine the accuracy of the position of the guide. In addition, the calculated movement helps in determining whether the patient and the region of interest is in the same position as during scan. Processing circuitry 90 is configured to determine breathing position of the patient during scan and to monitor the breathing position during an interventional procedure. The monitored breathing position is displayed to a physician performing the procedure.

In one embodiment, the movement sensing module 72 is a breath hold monitor. In the illustrated embodiment, the breath hold monitor is disposed on the torso of the patient. The breath hold monitor is configured to sense movement of a patient due to breathing. The movement is converted to corresponding digital signals that are processed to determine if the sensed movement if within a pre-defined limit. In one embodiment, the sensed movement can be displayed on a display unit (not shown). In one embodiment, patient movement restriction bed 78 can be used to restrict the movement of the patient.

When the sensed movement is within the pre-defined limit, the physician proceeds with the interventional procedure as planned. However, when the sensed movement is outside the pre-defined limit, the physician may choose to wait until the sensed movement is within acceptable limits before proceeding with the interventional procedure. In further embodiments, the tool positioning system further includes control circuitry (not shown) coupled to the processing circuitry that is configured to control movement of the robotic positioner based upon images created by the imaging system and/or movement caused due to breathing of the patient.

FIG. 4 is a diagrammatic view of the tool positioning system implemented according to aspects of the present technique. The various components of the tool positioning system are described in further detail below.

The tool positioning system includes two main components namely the standalone device 68 and the robotic positioner. The standalone device is mounted on a platform 80. In one embodiment, wheels 82 are attached to the base plate thereby enabling the tool positioning system to be moved from one position to another.. In one embodiment, the wheels 82 are of castor type which means that the wheels are mounted with an offset steering pivot such that the wheels will automatically swivel to align themselves in the direction where they are pushed. The wheels 82 allow easy movement of tool positioning system 66.

The robotic positioner 70 can be moved to position an interventional tool 78 at a desired position. In one embodiment, the end-effector 86 is configured to grip the interventional tool 78 using a gripper. The integrated computer 90 is configured to calculate the desired position. In one embodiment, a user, such as a physician, marks a target point and an entry point, on an image displayed on computer 90. These points are used to calculate a linear trajectory of the interventional tool. Using the linear trajectory, the desired coordinates to position the robotic positioner are calculated.

The tool positioning system is capable of motion in three linear directions that allows robotic positioner 70 to position interventional tool guide 88 to a desired point. Further, the robotic positioner provides linear motion along three mutually perpendicular axes and the end effector provides two angular degrees of freedom. Thus, the tool positioning system 66 is configured to accurately position the interventional tool with respect to patient's body by using the movement in these five axes.

The tool positioning system can be positioned near imaging system 20 and can be locked in the desired position. In one embodiment, the tool positioning system can be locked to a fixed docking plate which is described in further detail below.

FIG. 5A and FIG. 5B are diagrammatic views of a tool positioning system in an unlocked position and a locked position respectively. As can be seen in the figures, a docking plate 98 is fixed to the floor of the room. In one embodiment, the docking plate 98 is screwed to the pre-determined docking positions on the floor.

The tool positioning system is moved along direction 99 towards the docking plate 98. The tongue plate 84 on the bottom of the robotic position system 66 slides in to the docking plate 98 thereby getting locked into the position as shown in FIG. 5B.

FIG. 7 is a flow chart illustrating one method by which a robotic positioner can be used to position a tool guide at a desired position. The tool positioning system is docked adjacent to a patient cradle of an imaging system, such as a computed tomography system. Each system is described in further detail below.

At step 102, a patient is positioned on a patient cradle of the CT system. In one embodiment, the patient cradle further includes a patient movement restriction bed to limit any movement of the patient while performing various functions such as, for example, imaging, ablation and the like.

At step 104, a region of interest is scanned by the CT system. At step 106, a reference breath position of the patient is registered while scanning.

At step 108, the scanned images are transferred to the tool positioning system. In one embodiment, the images are displayed on a display unit integrated with the tool positioning system.

At step 110, an operator, such as a radiologist or a physician, marks an entry point and a target point on the image displayed on the tool positioning system. At step 112, a desired position is calculated based on the entry and target points. These desired position is converted to corresponding joint coordinates and cradle movement value. In a further embodiment, the display unit is configured to display the position of the robotic positioner with respect to the images, and the trajectory lines around the planned trajectory

At step 114, the tool guide and patient cradle is moved to the calculated spatial co-ordinates. In one embodiment, the robotic arm and the patient cradle are moved automatically.

At step 116, a breath hold position of the patient is determined. In one embodiment, a breath monitor is used to determine the breath position. If the breath hold position is outside a threshold value, the patient is instructed to hold breath until the threshold value is reached. When the breath position is within the desired threshold, the tool is inserted into the patient by the operator.

The above described techniques provide several advantages including the ability to perform the operation of inserting the tool when the patient is outside the imaging space of the imaging apparatus which decreases the risk of being exposed to unnecessary radiation. Also, the tool positioning system can be aligned with any existing imaging systems

While only certain features of the invention have been illustrated and described herein, many modifications and changes will occur to those skilled in the art. It is, therefore, to be understood that the appended claims are intended to cover all such modifications and changes as fall within the true spirit of the invention.

## Claims

1. A medical imaging system for conducting an image guided procedure on a subject, the medical imaging system comprising:
an imaging apparatus configured to generate images of a region of interest within the subject; wherein the medical imaging apparatus includes a movable patient cradle;
a tool positioning system configured to be moved to a desired position along the movable patient cradle of the imaging system and further configured to be referenced to the imaging system, the tool positioning system comprising:
a standalone device;
a robotic positioner coupled to the standalone device and configured to grip an interventional tool and move the tool to a desired position; and
a movement sensing module coupled to the standalone device and configured to calculate movement of the patient

2. The medical imaging system of claim 1, wherein the tool positioning system is disposed adjacent to the movable patient cradle at a pre-defined docking position.

3. The medical imaging system of claim 1, wherein the movement sensing module includes a breath sensor, preferably disposed on a torso of the patient.

4. The medical imaging system of claim 1, further comprising processing circuitry configured to determine the desired position based upon a plurality of parameters, preferably wherein the plurality of parameters include a position of the tool positioning system with respect to the movable patient cradle, length of the interventional tool and the pre-defined docking position, and optionally further comprising control circuitry coupled to the processing circuitry and configured to control movement of robotic positioner to the desired position..

5. The medical imaging system of claim 4, wherein pre-defined docking position is calculated based on a centre of an imaging space of the imaging apparatus, preferably wherein the pre-defined docking position is calculated during an installation operation of the standalone device.

6. The medical imaging system of claim 4, wherein the processing circuitry is configured to determine a distance to move the movable patient cradle such that the patient is outside of the imaging space of the imaging system while determining the desired position.

7. The medical imaging system of claim 1, further including a display device coupled to the standalone device and configured to display data related to the procedure

8. The medical imaging system of claim 1, wherein the robotic positioner has five axes of movement.

9. The medical system of in claim 1, wherein the imaging apparatus comprises a computed tomography (CT) system.

10. A tool positioning system adapted for use with an imaging system and for accurate positioning of a guide, the guide being suitable for inserting an interventional tool from an insertion point to a target point inside a patient, the system comprising:
a standalone device;
a robotic positioner coupled to the standalone device and configured to grip and move the guide to a desired position;
a movement sensing module coupled to the standalone device and configured to calculate movement of the patient from breathing; and
processing circuitry configured to determine the pre-defined docking position and the desired position, wherein the tool positioning system is configured to be docked at a pre-defined docking position along a movable patient cradle of the imaging system; wherein the standalone device is locked adjacent to the movable patient cradle

11. The tool positioning system of claim 10, wherein the desired position is calculated based on the insertion point and the target point, pre-defined docking position, coordinates derived from the location of the standalone device with respect to the patient cradle and a length of the interventional tool.

12. The tool positioning system of claim 10, wherein the processing circuitry is configured to determine a distance to move the movable patient cradle of the imaging system such that the patient is outside an imaging space of the imaging system while computing the position of the needle guide.

13. The tool positioning system of claim 10, further comprising control circuitry coupled to the processing circuitry and configured to control movement of the robotic positioner based upon images created by the imaging system.

14. The tool positioning system of claim 10, wherein the movement sensing module includes a breath hold monitor, preferably wherein the breath hold monitor is disposed on a torso of the patient.

15. The tool positioning system of claim 10, wherein the pre-defined docking position is computed based on the centre of an imaging space of the imaging apparatus.
